# EUROPEAN PATENT SPECIFICATION

(11) **EP 1 641 399 B1**
(45) Date of publication and mention of the grant of the patent: **19.09.2012**
(21) Application number: 04736892.3
(22) Date of filing: 15.06.2004
(51) Int. Cl.: A61B 17/00

(54) **WOUND CLOSURE AND SEALING DEVICE**
WUNDVERSCHLUSS- UND ABDICHTUNGSVORRICHTUNG
DISPOSITIF DE SUTURE DE PLAIE

(30) Priority: 03.07.2003 US 484310 P; 03.07.2003 SE 0301976
(43) Date of publication of application: 05.04.2006
(73) Proprietor: RADI MEDICAL SYSTEMS AB, 754 50 Uppsala (SE)
(72) Inventor: AKERFELDT, Dan, S-755 92 Uppsala (SE); MATHISEN, Torbjörn, S-125 42 Älvsjö (SE)
(74) Representative: Holmberg, Nils Anders Patrik
(86) International application number: PCT/SE2004/000938
(87) International publication number: WO 2005/002451

(56) References cited:
- EP-A1- 0 894 474
- WO-A1-98/31287
- US-A- 4 744 364
- US-A- 4 838 280
- US-A- 5 282 827
- US-A- 5 630 833
- US-A1- 2002 006 429

## Description

### Field of the invention

The present invention relates generally to medical sealing devices for sealing a percutaneous puncture in a wall of a vessel in a patient. The invention relates particularly to a sealing device comprising at least one sealing element, which is adapted to be positioned against the vessel wall for sealing the puncture hole therein, and an elongated member extending in a tissue canal leading from the patient's skin to the puncture hole in the vessel wall, which elongated member comprises a haemostatic material.

### Background of the invention

Several different types of devices and systems for sealing an opening or puncture in the wall of a blood vessel, duct or other lumen of a living being are known. So is, for example, a sealing device comprising a closure means adapted to be positioned against an inner surface of a vessel wall disclosed in US-4,744,364. This sealing device comprises further a retraction means, which is attached to the closure means and extends in an incision canal leading from a patient's skin to the puncture hole in the vessel wall. The retraction means, which can be in the form of a filament, holds the closure means in engagement with an inner surface of the vessel wall to thereby seal the puncture hole.

US-5,620,461 shows another sealing device comprising a sealing element adapted to be positioned at an inner surface of a vessel wall, a thread-like member attached to the sealing element, and an arresting element, which can move along the thread-like member into engagement with an outer surface of the vessel wall.

A common feature of the sealing devices disclosed in US-4,744,364 and 5,620,461, respectively, is that the actual sealing of a puncture hole in a vessel wall is accomplished from the inside of the vessel. Other sealing devices wherein the sealing mainly is accomplished from the inside of a vessel are disclosed in US patent 6,508,828 as well as in US applications 09/836,529 and 10/280,086, which all are assigned to the present assignee.

In US-5,282,827 a principally different solution is presented in that the sealing instead is performed at the outside of a vessel wall. Here, a sealing device comprises an anchor adapted to be positioned at an inner surface of a vessel wall, a compressed collagen plug, and a filament connecting the anchor and the collagen plug. In use, the collagen plug is expelled from a carrier and is moved into engagement with the outside of the vessel wall, while the filament is left in a tract leading to the puncture hole.

Other devices, such as the devices disclosed in US-5,342,393 and EP-474,752-B2, respectively, rely on the combination of an inner member and an outer member to seal a puncture hole in a vessel wall. In this case, the sealing is achieved by clamping together the inner and other members, with the vessel wall being disposed therebetween.

US-6,325,789 proposes another method for sealing a puncture hole in a vessel wall. According to this invention, a charge of haemostatic material, which can include collagen, fibrin glue or thrombin, is placed in a tissue canal leading to the puncture hole in the vessel wall. In contrast to, for example, the device disclosed in the above-mentioned US-5,282,827, the haemostatic material performs all the sealing, without the assistance of any anchoring member being positioned within the vessel.

The common object of the devices and methods according to the documents listed above is to prevent bleeding through a puncture hole in a wall of a blood vessel. No measures are, however, taken to prevent or reduce the bleeding that can appear in an incision canal leading to the puncture hole. Consequently, there is a need in the art for an improved device for sealing a puncture hole in the wall of a blood vessel, the puncture hole being the result of a percutaneous operation that has created an incision canal extending from the skin of a patient to the puncture hole.

### Summary of the invention

An object of the present invention is to provide an improved device for sealing a puncture hole in a wall of a blood vessel, the improvement being that the device not only seals the puncture hole itself, such that the primary bleeding is stopped, but also prevents that a secondary bleeding appears in an incision canal leading from the skin of a patient to the puncture hole in the blood vessel wall.

Generally, a sealing device according to the present invention belongs to a class of closure devices wherein a sealing device comprises at least one sealing element adapted to be positioned against a wall of a vessel to seal a puncture hole therein, and an elongated member, which is attached to the sealing element and which, after the sealing element has been positioned against the vessel wall, extends in an incision canal leading to the puncture hole. According to the invention, the elongated member includes a haemostatic material that stops or at least minimizes bleeding that occurs in the incision canal itself.

In one embodiment of the invention, the elongated member is a suture or filament, which has been coated or otherwise provided with a haemostatic material. The haemostatic material can be in the form of a clotting agent, such a thrombin, which promotes blood clotting in the incision canal to thereby prevent so-called oozing in the incision canal.

A haemostatic material in the elongated member will also be valuable in those cases where the sealing device does not function properly, something that may happen when the sealing element has not been correctly positioned against a blood vessel wall, or when the sealing device malfunctions for some other reason, such that blood from the vessel leaks into the incision canal. Another object of the present invention is therefore to provide a sealing device for sealing a percutaneous puncture that has created an incision canal extending from a patient's skin, through tissue disposed between the skin and a vessel, and through a wall of the vessel, comprising at least one sealing element adapted to be positioned against the vessel wall to thereby seal the puncture hole therein, and an elongated member, which is connected to the sealing element and which is adapted to extend in the incision canal, the elongated member comprising a haemostatic material that promotes the sealing of the incision canal.

### Brief description of the drawings

Fig. 1 is a schematic illustration of a first embodiment of the present invention.
Fig. 2 is a schematic illustration of a second embodiment of the present invention.
Fig. 3 is a schematic illustration of a third embodiment of the present invention.
Fig. 4 is a schematic illustration of a fourth embodiment of the present invention.
Fig. 5 is a schematic illustration of an elongated member according to the present invention.
Fig. 6 is a first example of a cross-section of an elongated member according to the present invention.
Fig. 7 is a second example of a cross-section of an elongated member according to the present invention
Fig. 8 is a third example of a cross-section of an elongated member according to the present invention.

### Detailed description of the preferred embodiments

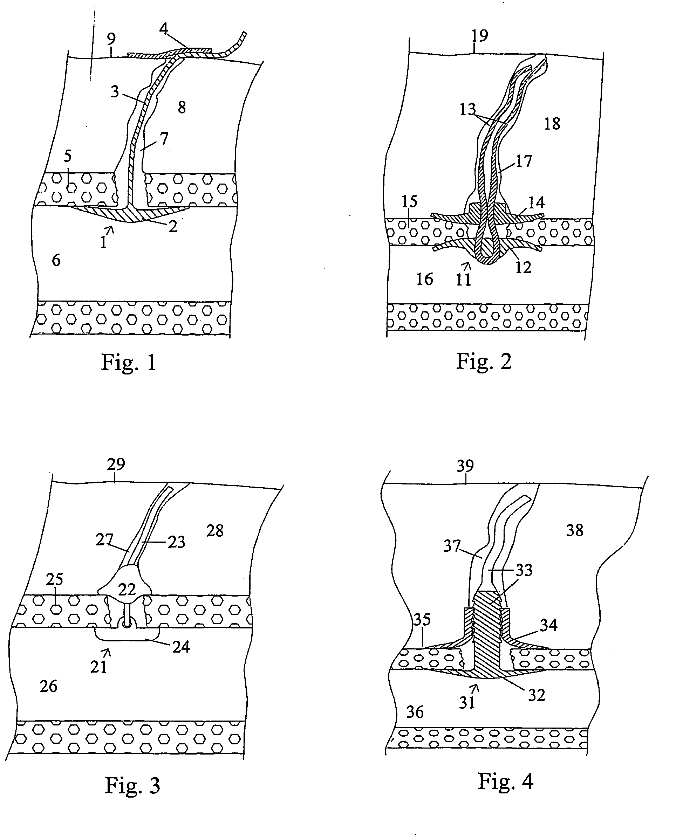

As stated above, the present invention provides an improved sealing device comprising at least one sealing element, which is adapted to be positioned against a wall of a blood vessel to stop bleeding through a puncture hole in the blood vessel, and an elongated member, which is connected to the sealing element and adapted to be positioned in a tissue canal going through a patient's skin, through tissue overlaying a vessel, and through a wall of the vessel. The basic configuration of such a sealing device can be similar to various sealing devices known in the art, as long as the sealing device comprises at least one sealing element and an elongated member adapted to extend in a tissue canal. Examples of different embodiments of the present sealing device will be given below in conjunction with Fig. 1 to Fig. 4. According to the invention, the elongated member includes a haemostatic material that prevents or reduces secondary bleeding in or into the incision canal. Herein, the term "secondary bleeding" includes diffuse bleeding, also referred to as oozing, which does not emanate from a specific blood vessel as well as bleeding from smaller blood vessels that have been damaged during the percutaneous operation that created the incision canal. Furthermore, the term "secondary bleeding" is also intended to include bleeding from the blood vessel itself, whereby such bleeding, for example, may be the result of an improperly positioned sealing device.

Fig. 1 illustrates a first embodiment of a sealing device 1 according to the present invention. The sealing device 1 comprises a sealing element 2 and an elongated member 3, which is attached to the sealing element 2. In use, the sealing element 2 is positioned against an inner surface of a wall 5 of a vessel 6, while the elongated member 3 is positioned in an incision canal 7 extending from the vessel wall 5, through tissue 8 and to the skin 9 of a patient. In this embodiment, a piece of adhesive tape 4 has been attached to the skin 9 and to an end portion of the elongated member 3 in order to hold the elongated member 3 taut, thereby holding the sealing element 2 in sealing engagement with the inner surface of the vessel wall 5. According to the invention, the elongated member 3, which can be in the form of a suture or filament, comprises a haemostatic material that stops secondary bleeding in the incision canal 7. Examples of different suitable haemostatic materials as well as examples of how such materials can be incorporated in an elongated member will be given below.

In Fig. 2 a second embodiment of a sealing device 11 according to the invention is shown. The sealing device 11 comprises a sealing element 12, an elongated member 13 connected to the sealing element 12, and a locking element 14. The figure illustrates that the sealing element 12 is positioned against an inner surface of a wall 15 of a vessel 16, with the elongated member 13 being positioned in an incision canal 17 extending from the vessel wall 15, through tissue 18 and to the skin 19 of a patient. A comparison between Fig. 1 and Fig 2 reveals that the adhesive tape 4 of Fig. 1 in the second embodiment shown Fig. 2 has been replaced with the locking element 14. The locking element 14 can slide along the elongated member 13 into contact with an outer surface of the wall 15 of the vessel 16, where the locking element 14 is held in place by friction locking. Also in this case the sealing is accomplished from the inside of the vessel 16, i.e. by the sealing element 12 which is held in engagement with an inner surface of the vessel wall 15 by the locking element 14 and the elongated member 13. In this embodiment, the elongated member 13 is in the form of a pair of sutures or filaments, of which at least one comprises a suitable haemostatic material. Here, it should be noted that this second embodiment of a sealing device is intended to encompass both sealing devices wherein an outer locking element only serves to hold the inner sealing element in place, i.e. when the inner sealing element performs essentially all the sealing and the locking element acts as a true locking member, as well as sealing devices wherein the sealing is accomplished by clamping the vessel wall between inner and outer members. In the latter case, the term "locking element" should therefore not be literally interpreted.

A third embodiment of a sealing device 21 according to the invention is illustrated in Fig. 3. The sealing device 21 comprises a sealing element 22, an elongated member 23, and an anchoring member 24 attached to the elongated member 23. In this embodiment, the sealing element 22 is in the form of plug 22 made from a suitable material such as collagen. In use, the plug 22 is pushed into position against an outer surface of a wall 25 of a vessel 26, with the elongated member 23 being positioned in an incision canal 27 extending from the vessel wall 25, through the sealing element 22 and through tissue 28, and to the skin 29 of a patient. As should be apparent from the figure, the sealing is in this case performed at the outside of the vessel 26, with the anchoring member 24 mainly acting as an anchor for the elongated member 23 to thereby hold the elongated member 23 and the sealing element 22 in place. Also here the elongated member 23, which can be in the form of a suture or a filament, comprises a suitable haemostatic material. Further, it should be noted that a sealing element, here a collagen plug 22, can be held in place by means of an elongated member without the sealing element being fixedly attached to the elongated member.

Fig. 4 is a schematic illustration of a fourth embodiment of the present invention. Here, a sealing device 31 comprises a first sealing element 32 and an elongated member 33, which extends from the first sealing element 32 and through a second sealing element 34. A portion of the elongated member 33 has been provided with saw-teeth that fit into corresponding saw-teeth in the second sealing element 34. In use, the first sealing element 32 is positioned against an inner surface of a wall 35 of a vessel 36. Thereafter, the second sealing element 34 is pushed over the elongated member 33 until the vessel wall 35 is clamped between the first and second sealing elements 32, 34, which are held in place by the saw-teeth provided on the elongated member 33 and the second sealing element 34, respectively. In this position, a proximal portion of the elongated member 33 extends in an incision canal 37 leading from the vessel wall 35, through tissue 38 and to the skin 39 of a patient. According to the invention, the proximal portion of the elongated member 33 includes a haemostatic material that stops or minimizes secondary bleeding in the incision canal 37.

For those types of sealing devices that include a second element which is adapted to be positioned at an outer surface of a vessel wall, it is conceivable that also this second element comprises a haemostatic material, which promotes sealing of the puncture hole in the vessel wall. This feature has explicitly been mentioned for the sealing element 22 of Fig. 3, which could be made from collagen, but also the locking element 14 of Fig. 2 as well as the second sealing element 34 of Fig. 4 could comprise a haemostatic material, substance or agent as a complement to a haemostatic elongated member.

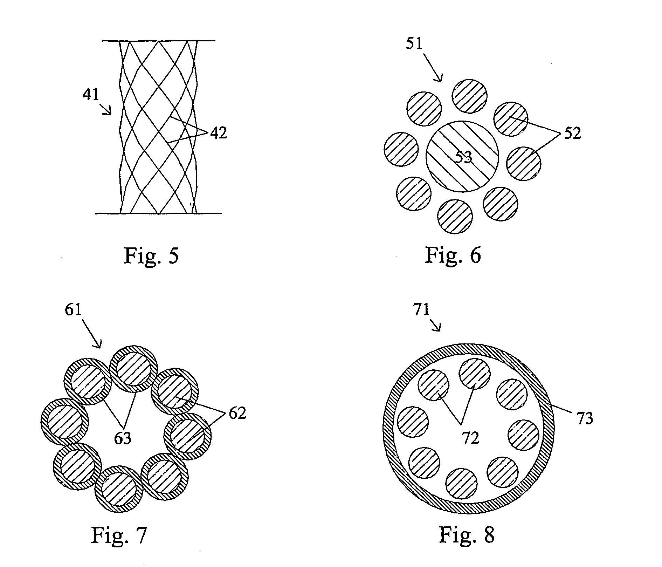

Above, several different embodiments of those elements of a sealing device that seal a puncture hole in a vessel wall have been described in conjunction with Fig. 1 to Fig. 4, while relatively little have been said about the elongated members which, according to the invention, provide the sealing of an incision canal leading to the puncture hole. An elongated member can be in the form one or several sutures, filaments or multifilaments, which have been provided with a haemostatic material. The general design of such an elongated member 41 is illustrated in Fig. 5. The elongated member 41 comprises several threads or filaments 42, which have been joined together by means of a suitable technique, such as spinning, twining, braiding, etc.

In Fig. 6 is illustrated a first example of a cross-section of an elongated member 51, in this case a multifilament 51. The multifilament 51 comprises several relatively thin filaments or threads 52 surrounding a haemostatic core 53. The haemostatic core 53 contains or is made from one or several suitable haemostatic materials, examples of which will be given below.

Another example of how an elongated member in the form of a multifilament can be provided with a haemostatic material is illustrated in Fig. 7. Here a multifilament 61 comprises several filaments 62, each of which has been coated with a separate layer 63 of a haemostatic material.

Instead of coating each filament of a multifilament with a haemostatic material, the whole multifilament can be coated with a layer of haemostatic material. An example of such an embodiment is shown in Fig. 8, where a multifilament made up of several filaments 72 constitutes the core of an elongated member 71 that further comprises a coating 73 of a haemostatic material.

Here it should be apparent that an elongated member according to the present invention can assume various shapes, and the haemostatic material can be incorporated in a number of ways. It is, for example, conceivable that an elongated member is impregnated or soaked with a suitable haemostatic material, or the haemostatic material can be contained in a separate elongated capsule that may constitute the core of the elongated member. Further, the elongated member can be provided as a flexible stem extending from an inner (or outer) seal, and the elongated member can be made from any materials, such as biodegradable materials, known in the art, as long as such materials can be provided with a haemostatic component.

The cross-sectional diameter of the elongated member, being a suture, filament, multifilamet or a flexible stem, is small in comparison to the diameter of the inner (or outer) sealing element 2, 12, 14, 22, 24, 32, 34 taken in the plane perpendicular to the drawing papers. By small is herein meant less than 25% and preferably less than 10%. The sealing element may have a variety of physical geometrical shapes in the plane perpendicular to the plane of the illustrated drawings. They could e.g. have a circular, elliptical or rectangular shape. Herein is meant by "diameter" the longest distance across the sealing element.
One typical sealing element may have an elliptical shape with the axes 10 mm and 5 mm and a typical diameter of an elongated member may be in the range of 0,2-2 mm. If the elongated member is a suture it has a diameter of approximately 0,3 mm.

According to the present invention, an elongated member can be provided with a variety of different haemostatic materials and substances. Some of these materials and substances performs to the haemostasis by mechanically stop bleeding into an incision canal, i.e, these materials swell in contact with a body fluid such that an elongated member being provided with such a material occupies the available space within the incision canal and thereby prevents blood or other fluids from entering into the incision canal. Other haemostatic agents promote the clotting of the blood, while still other haemostatic agents cause vasoconstriction. Non-limiting examples of haemostatic agents that can be used in an elongated member according to the invention are: collagen, chitin and chitosan, thrombin, gelatine, oxidized regenerated cellulose, aprotinin, tranexamic acid, aminocaproic acid, desmopressin, vitamin K, factor VIIa, factor VIII, vasopressin, and conjugated oestrogen, or combinations thereof.

Although the present invention has been described with reference to specific embodiments, also shown in the appended drawings, it will be apparent for those skilled in the art that many variations and modifications can be done within the scope of the invention as described in the specification and defined with reference to the following claims. In particular, it can be noted that the present invention is applicable on other vessels than blood vessels, and that the invention is not limited to any special kind of haemostatic material, substance or agent.

## Claims

1. Device (1; 11 21; 31) for sealing a puncture in a vessel comprising a seating element (2; 12; 22; 32) configured to be placed against a wall of the vessel and to seal the puncture in the vessel by contacting the vessel wall, and an elongated member (3; 13; 23; 33) connected to the sealing element and configured to extend in an incision canal leading to the puncture in the vessel, wherein the elongated member has a diameter that is small, less than 25%, preferably less than 10%, in comparison to the diameter of the sealing element and comprises a haemostatic material, **characterized in that** the haemostatic material is a core of the elongated member, or that the elongated member is coated, impregnated or soaked with the haemostatic material.

2. Device (1) according to claim 1, **characterized in that** the elongated member at least partly is in the form of a suture_{;} filament or multifilament.

3. Device (1) according to claim 1, **characterized in that** the sealing element (2) is adapted to be positioned against an inner surface of the vessel wall and is held in place by the elongated member (3).

4. Device (11) according to claim 1, **characterized in that** the sealing element (12) is adapted to be positioned against an inner surface of the vessel and that the device (11) further comprises a locking element (14) connected to the elongated member (13) and adapted to be positioned against an outer surface of the vessel wall.

5. Device (11) according to claim 4, **characterized in that** the locking element (14) comprises a haemostatic material.

6. Device (21) according to claim 1, **characterized in that** the sealing element (22) is in the form of plug (22), which is adapted to be positioned against an outer surface of the vessel wall, and that the device (21) further comprises an anchor member (24) connected to the elongated member (23) and adapted to be positioned against an inner surface of the vessel wall.

7. Device (21) according to claim 5, **characterized in that** the plug (22) comprises a haemostatic materials.

8. Device (31) according to claim 1, **characterized in that** the sealing element (32) is adapted to be positioned against an inner surface of the vessel wall, and that the device (31) further comprises a second scaling element (34), which is adapted to be positioned against an outer surface of the vessel wall and is provided with saw-teeth that fit into corresponding recesses provided on a portion of the elongated member (33) that extends through the second sealing element (34).

9. Device (31) according to claim 8, **characterized in that** the second sealing element (34) comprises a haemostatic material.

10. Device according to anyone of the claims 1 to 9, **characterized in that** the elongated member is a multifilament comprising several filaments, each of which is coated with the haemostatic material.

11. Device according to anyone of the claims 1 to 10, **characterized in that** the haemostatic material is selected from the group comprising collagen, chitin and chitosan, thrombin, gelatine, oxidized regenerated cellulose, aprotinin, tranexamic acid, aminocaproic acid, desmopressin, vitamin K, factor VIIa, factor VIII, vasopressin, and conjugated oestrogen, or combinations thereof.

## Patentansprüche

1. Vorrichtung (1; 11; 21; 31) zum Abdichten einer Einstichstelle in einem Gefäß, die ein Abdichtungselement (2; 12; 22; 32) umfasst, das dazu eingerichtet ist, gegen eine Wand des Gefäßes platziert zu werden und die Einstichstelle im Gefäß durch Kontakt mit der Gefäßwand abzudichten, und ein langgestrecktes Element (3; 13; 23; 33), das mit dem Abdichtungselement verbunden ist und eingerichtet ist, sich in einen Einschnittkanal, der zum Einstich in das Gefäß führt, zu erstrecken, worin das langgestreckte Element einen kleinen Durchmesser, weniger als 25%, vorzugsweise weniger als 10% im Vergleich zum Durchmesser des Abdichtungselements aufweist und ein blutstillendes Material umfasst, **dadurch gekennzeichnet, dass** das blutstillende Material ein Kern des langgestreckten Elements ist, oder dass das langgestreckte Element mit dem blutstillenden Material beschichtet, imprägniert oder getränkt ist.

2. Vorrichtung (1) gemäß Anspruch 1, **dadurch gekennzeichnet, dass** das langgestreckte Element zumindest teilweise in Form einer Naht, eines Filaments oder eines Multifilaments vorliegt.

3. Vorrichtung (1) gemäß Anspruch 1, **dadurch gekennzeichnet, dass** das Abdichtungselement (2) dafür eingerichtet ist, gegen eine innere Oberfläche der Gefäßwand positioniert zu werden und durch das langgestreckte Element (3) am Platz gehalten wird.

4. Vorrichtung (11) gemäß Anspruch 1, **dadurch gekennzeichnet, dass** das Abdichtungselement (12) dafür eingerichtet ist, gegen eine innere Oberfläche der Gefäßwand positioniert zu werden, und dass die Vorrichtung (11) weiterhin ein Schließelement (14) umfasst, dass mit dem langgestreckten Element (13) verbunden ist und dafür eingerichtet ist, gegen eine äußere Oberfläche der Gefäßwand positioniert zu werden.

5. Vorrichtung (11) gemäß Anspruch 4, **dadurch gekennzeichnet, dass** das Schließelement (14) weiterhin ein blutstillendes Material umfasst.

6. Vorrichtung (21) gemäß Anspruch 1, **dadurch gekennzeichnet, dass** das Abdichtungselement (22) in Form eines Stöpsels (22) vorliegt, der dafür eingerichtet ist, gegen eine äußere Oberfläche der Gefäßwand positioniert zu werden, und dass die Vorrichtung (21) weiterhin ein Ankerelement (24) umfasst, das mit dem langgestreckten Element (23) verbunden ist und dafür eingerichtet ist, gegen eine innere Oberfläche der Gefäßwand positioniert zu werden.

7. Vorrichtung (21) gemäß Anspruch 5, **dadurch gekennzeichnet, dass** der Stöpsel (22) ein blutstillendes Material umfasst.

8. Vorrichtung (31) gemäß Anspruch 1, **dadurch gekennzeichnet, dass** das Abdichtungselement (32) dafür eingerichtet ist, gegen eine innere Oberfläche der Gefäßwand positioniert zu werden, und dass die Vorrichtung (31) weiterhin ein zweites Abdichtungselement (34) umfasst, das dafür eingerichtet ist, gegen eine äußere Oberfläche der Blutgefäßwand positioniert zu werden und das mit Sägezähnen versehen ist, die in entsprechende Vertiefungen passen, die auf einem Abschnitt des langgestreckten Elements (33) angebracht sind, das sich durch das zweite Abdichtungselement (34) erstreckt.

9. Vorrichtung (31) gemäß Anspruch 8, **dadurch gekennzeichnet, dass** das zweite Abdichtungselement (34) ein blutstillendes Material umfasst.

10. Vorrichtung gemäß irgendeinem der Ansprüche 1 bis 9, **dadurch gekennzeichnet, dass** das langgestreckte Element ein Multifilament ist, das mehrere Filamente umfasst, von denen jedes mit dem blutstillenden Material beschichtet ist.

11. Vorrichtung gemäß irgendeinem der Ansprüche 1 bis 10, **dadurch gekennzeichnet, dass** das blutstillende Material aus der Gruppe bestehend aus Collagen, Chitin und Chitosan, Thrombin, Gelatine, oxidierte, regenerierte Cellulose, Aprotinin, Tranexamsäure, Aminocapronsäure, Desmopressin, Vitamin K, Faktor VIIa, Faktor VIII, Vasopressin und konjugiertes Östrogen oder Kombinationen davon ausgewählt ist.

## Revendications

1. Dispositif (1; 11 ; 21 ; 31) destiné à fermer une ponction dans un vaisseau, comprenant un élément de fermeture (2 ; 12 ; 22 ; 32) configuré de manière à être placé contre une paroi du vaisseau, et à fermer la ponction dans le vaisseau par une mise en contact avec la paroi du vaisseau, et un élément allongé (3 ; 13 ; 23 ; 33) relié à l'élément de fermeture et configuré de manière à s'étendre dans un canal d'incision conduisant à la ponction dans le vaisseau, dans lequel l'élément allongé présente un diamètre faible, inférieur à 25 %, de préférence inférieur à 10 %, du diamètre de l'élément de fermeture et comprend un matériau hémostatique, **caractérisé en ce que** le matériau hémostatique est un noyau de l'élément allongé, ou **en ce que** l'élément allongé est enduit, imprégné ou imbibé avec le matériau hémostatique.

2. Dispositif (1) selon la revendication 1, **caractérisé en ce que** l'élément allongé est au moins partiellement de la forme d'un filament de suture ou d'un multifilament de suture.

3. Dispositif (1) selon la revendication 1, **caractérisé en ce que** l'élément de fermeture (2) est apte à être positionné contre une surface interne de la paroi du vaisseau et est maintenu en place par l'élément allongé (3).

4. Dispositif (11) selon la revendication 1, **caractérisé en ce que** l'élément de fermeture (12) est apte à être positionné contre une surface interne de la paroi du vaisseau et **en ce que** le dispositif (11) comprend en outre un élément de verrouillage (14) relié à l'élément allongé (13) et apte à être positionné contre une surface externe de la paroi du vaisseau.

5. Dispositif (11) selon la revendication 4, **caractérisé en ce que** l'élément de verrouillage (14) comprend un matériau hémostatique.

6. Dispositif (21) selon la revendication 1, **caractérisé en ce que** l'élément de fermeture (22) est de la forme d'un bouchon (22), lequel est apte à être positionné contre une surface externe de la paroi du vaisseau, et **en ce que** le dispositif (21) comprend en outre un élément d'ancrage (24) relié à l'élément allongé (23) et apte à être positionné contre une surface interne de la paroi du vaisseau.

7. Dispositif (21) selon la revendication 5, **caractérisé en ce que** le bouchon (22) comprend un matériau hémostatique.

8. Dispositif (31) selon la revendication 1, **caractérisé en ce que** l'élément de fermeture (32) est apte à être positionné contre une surface interne de la paroi du vaisseau, et **en ce que** le dispositif (31) comprend en outre un second élément de fermeture (34), lequel est apte à être positionné contre une surface externe de la paroi du vaisseau et est pourvu de dents de scie qui s'insèrent dans des cavités correspondantes délivrées sur une partie de l'élément allongé (33) qui s'étend à travers le second élément de fermeture (34).

9. Dispositif (31) selon la revendication 8, **caractérisé en ce que** le second élément de fermeture (34) comprend un matériau hémostatique.

10. Dispositif selon l'une quelconque des revendications 1 à 9, **caractérisé en ce que** l'élément allongé est un multifilament comprenant plusieurs filaments, dont chacun est enduit du matériau hémostatique.

11. Dispositif selon l'une quelconque des revendications 1 à 10, **caractérisé en ce que** le matériau hémostatique est sélectionné dans le groupe comprenant : collagène, chitine et chitosane, thrombine, gélatine, cellulose régénérée oxydée, aprotinine, acide tranexamique, acide aminocaproïque, desmopressine, vitamine K, facteur VIIa, facteur VIII, vasopressine, et oestrogène conjugué, ou combinaisons de ceux-ci.
